(19) Europäisches Patentamt
European Patent Office
Office européen des brevets

(11) Publication number : **0 593 284 A1**

(12) **EUROPEAN PATENT APPLICATION**

(21) Application number : **93308174.7**

(51) Int. Cl.[5] : **C08G 65/32,** A61L 27/00

(22) Date of filing : **13.10.93**

(30) Priority : **13.10.92 US 960669**

(43) Date of publication of application :
**20.04.94 Bulletin 94/16**

(84) Designated Contracting States :
**CH DE DK FR GB IT LI NL SE**

(71) Applicant : **SIEMENS AKTIENGESELLSCHAFT**
**St Martin Strasse 76**
**D-81541 München 2 (DE)**

(72) Inventor : **Vachon, David J.**
**16107 Harvest Street**
**Granada Hills, CA 91344 (US)**

(74) Representative : **Rees, David Christopher et al**
**Kilburn & Strode 30 John Street**
**London WC1N 2DD (GB)**

(54) **Derivatised polyoxyalkylene anti-fouling coatings for medical devices.**

(57) Coatings for medical devices which prevent the fouling of such devices when implanted in mammalian bodies are prepared from polymers comprising at least one poly(alkylene oxide) segment having at least 2 repeating $-R_1-O-$ groups, where $R_1$ is an alkyl group containing at least 1 carbon atom, the polymer having two terminal photochemically or thermochemically reactive moieties. The average molecular weight of the polymer is at least 10,000. The polymer is used to coat the medical device and is then cured by exposure to ultraviolet light or by heat. The resultant polymeric coating is tenacious, lubricous, hydrophilic and resists fouling of the medical device. Biodegradable coatings can be prepared by incorporating amino acids and/or peptides into the polymer backbone. The polymer can be linear or nonlinear. Block co-polymers can be used, the block copolymer being prepared by utilizing precursors containing more than one type of alkyl group.

EP 0 593 284 A1

This invention relates to methods and compounds for modifying the biocompatibility of the surfaces of medical devices to prevent fouling of these devices.

The development of implantable synthetic medical devices, such as artificial blood vessels, catheters, pacemakers, prosthetic implants, and drug delivery systems for the controlled release of therapeutics, represent a small fraction of material-dependent devices in a rapidly developing area. However, the long-term use of these implantable devices can be impaired by thrombosis, infection, as well as chronic inflammation, which can cause fouling of these devices. The fouling can result in a decrease in device performance and longevity.

Proteins deposited from the blood can initiate deposition of the cellular components within the blood onto the surface of the medical devices. This deposition constricts and hinders the use of devices such as artificial blood vessels. Inflammation, a natural response to an implant, can also initiate reactions that impede the function of devices such as ophthalmic lenses and pacemakers leads. Consequently, it is highly desirable to prevent such fouling of biomaterial surfaces.

Fouling of a medical device can be prevented by modifying the surface of the device to inhibit fouling. Many different materials can be used to limit and inhibit fouling. One such material is polyethylene oxide (PEO). However, there are several problems associated with PEO coatings.

A major problem with PEO is its high solubility in water. This necessitates the use of complex and expensive methods allowing the utilization of PEO as an anti-fouling coating material. These complex techniques include such methods as: covalent grafting; plasma polymerization; preparation of polymers containing pendant groups; preparation of block co-polymers; and the absorption of surfactants into a polymeric surface. The complexity of these techniques raises the level of difficulty in controlling coating uniformity, thickness and consistency.

Furthermore, many of these reactions use catalysts and/or reaction initiators that result in undesirable reaction byproducts which have to be subsequently removed by separation techniques. For example, U.S. Patent No. 4,177,056 describes the use of water insoluble PEO's terminated with bismethacrylates. These require a free radical initiator for polymerization, resulting in undesirable byproduct contaminants within the coating.

Thus, many current techniques of applying surface modifiers are disadvantageous because of their complexity, cost, lack of consistency, uniformity and reduced effectiveness in protein repulsion.

Accordingly, there is a need for non-toxic compositions and methods of applying these compositions to medical devices to render surfaces of the medical devices biocompatible, where the compositions and methods are simple and provide consistent and uniform thicknesses.

The present invention provides compositions of matter and methods for their application that satisfy these needs. Coatings prepared in accordance with the present invention have high anti-fouling characteristics and have excellent tenacity and lubricity. Furthermore, the method of affixing the compositions onto the surfaces of medical devices is simple, results in uniform and consistent coatings, and does not use added catalysts or reaction initiators.

A method suitable for preparing these coatings comprises the following steps: 1) coating at least a portion of a medical device with an aqueous solution of a photochemically reactive or thermochemically reactive polymer comprising at least one linear poly(alkylene oxide) segment having at least two repeating alkylene oxide groups, substantially all of the polymer having at least two terminal photochemically reactive or thermochemically reactive groups, the molecular weight of the polymer being at least 10,000 and sufficiently low that the polymer is soluble in water; and 2) curing the coating by exposing the coating to ultraviolet light in case of a photochemically reactive polymer or by heating the polymer in the case of a thermochemically reactive polymer.

By the term "poly(alkylene) oxide segment" it is meant a segment or unit having the structure

$$-\left[R - O\right]_n-$$

where $R_1$ is an alkyl moiety containing at least 1 carbon atom and n is at least 2; typically n is at least 16.

The reactive polymer used for these coatings typically is derived from an aliphatic poly(ether glycol), referred to herein as a poly(alkylene oxide), such as poly(ethylene oxide) or poly(ethylene glycol). It must be understood that poly(alkylene oxides) and poly(alkylene glycols) are both hydroxy terminated aliphatic polyethers, the only difference being in molecular weight. Polymers with molecular weights of less than 10,000 are referred to herein as poly(alkylene glycols), and those with molecular weights of 10,000 and above are referred to as poly(alkylene oxides).

The formula of the reactive polymer depends on the structure of the original polymer and the type of photochemically reactive group that is used. When a hydroxy terminated alkylene oxide polymer is used, the reactive polymer has the formula:

$$(1)\qquad P_1 - O - \left[ R_1 - O \right]_n R_1 - O - P_1$$

When an amine terminated poly(alkylene oxide) is used, the reactive polymer has the formula:

$$(2)\qquad P_1 - \overset{R4}{\overset{|}{N}} - R_1 - O - \left[ R_1 - O \right]_n \overset{R4}{\overset{|}{N}} - P_1$$

When penta-erythritol is used as building block, the reactive polymer can have one of the formulas:

$$(3)\qquad C - \left[ - CH_2 - O - \left( - R_1 - O - \right)_n - R_1 - O - P_1 \right]_4$$

$$(4)\qquad C - \left[ - CH_2 - \underset{R_4}{\underset{|}{N}} - R_1 - \left( - O - R_1 - \right)_n - O - R_1 - \underset{R_4}{\underset{|}{N}} - P_1 \right]_4$$

When a diethanol amine or triethanol amine is used as a building block, the reactive polymer has the formula:

$$(5)\qquad R_5 - N - \left[ - R_6 - O - \left( - R_1 - O - \right)_n - R_1 - O - P_1 \right]_x$$

In formulas 1-5:

$R_1$ is an alkyl group containing at least one carbon atom and typically 2 to 10 carbon atoms;

n is at least 1 and sufficiently large that the average molecular weight of the polymer is at least 10,000, and preferably about 20,000;

$R_4$ is selected from the group consisting of hydrogen, hydrocarbon groups containing up to about 10 carbon atoms, and heteroatom-containing hydrocarbon groups containing up to about 10 carbon atoms;

$R_6$ is an alkyl group containing up to 10 carbon atoms, and typically 2 carbon atoms;

where x is 2 or 3, and for x of 2, $R_6$ is selected from the group consisting of hydrogen, hydrocarbon groups containing up to about 10 carbon atoms, and heteroatom-containing hydrocarbon groups containing up to about 10 carbon atoms, and for x of 3, $R_6$ is excluded;

$P_1$ is a photochemically reactive or thermochemically reactive moiety capable of undergoing crosslinking on photoactivation or heating respectively, to form a hydrophilic, lubricous and tenacious coating which resists fouling.

Each $P_1$ can be the same or different, but for simplicity, the $P_1$'s are the same. Preferably $P_1$ is photochemically reactive.

The term "heteroatom-containing hydrocarbon group" refers to a hydrocarbon group that also contains a heteroatom such as oxygen, nitrogen, or sulfur.

Preferably $R_4$ is hydrogen or a lower alkyl group having from 1 to about 6 carbon atoms, and most preferably is hydrogen or methyl.

The base polymer can also have different alkyl groups in blocks within its structure, such that the photochemically reactive polymer, has one of the formulas:

$$(6) \quad P_1 - O - R_1 - \left[ O - R_1 \right]_m - O - \left[ R_2 - O \right]_n - P_1$$

$$(7) \quad P_1 - \underset{\displaystyle |}{\overset{R_4}{N}} - R_1 - \left[ O - R_1 \right]_m - O - \left[ R_2 - O \right]_n - R_2 - \overset{R_4}{N} - P_1$$

$$(8) \quad C-\left[ -CH_2-O-\left( -R_1-O- \right)_m -R_1-O-\left( -R_2-O- \right)_n -R_2-O-P_1 \right]_4$$

$$(9) \quad C-\left[ -CH_2-\underset{R_4}{N}-R_1-\left( -O-R_1- \right)_m -O-\left( -R_2-O- \right)_n -R_2-\underset{R_4}{N}-P_1 \right]_4$$

$$(10) \quad R_5-N-\left[ -R_6-O-\left( -R_1-O- \right)_m -R_1-O-\left( -R_2-O- \right)_n -R_2-O-P_1 \right]_x$$

In formulas 6-10:

$R_1$ and $R_2$ are alkyl groups containing at least one carbon atom, and typically 2 to 10 carbon atoms, $R_1$ is different from $R_2$;

$P_1$, $R_4$, $R_5$, $R_6$ and x are as defined above; and

m and n are at least 1, and m and n are sufficiently large that the average molecular weight of the polymer is at least 10,000.

The photochemically reactive group typically can be a substituted benzophenone or an acidonitrophenyl group. These respectively have the structures:

(11)

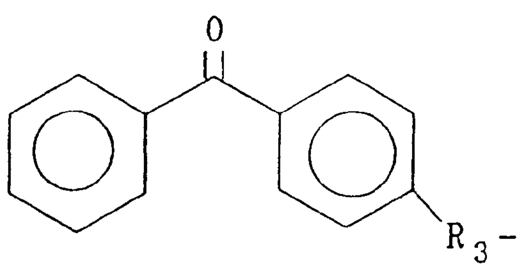

where $R_3$ is an alkyl or heteroatom-containing alkyl group having from 1 to about 6 carbon atoms, as well as acyl groups, or alkyl or heteroatom-containing alkyl groups with acyl termini.

(12) 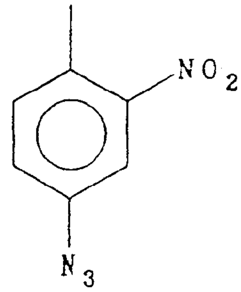

For the preparation of biodegradable coatings, it is desirable to include in the polymer backbone a moiety that renders the coating biodegradable. A polymer containing such a moiety can have one of the following formulas:

(13) $$P_1 - Y - O - \left[ R_1 - O \right]_n - R_1 - O - Y - P_1$$

(14) $$P_1 - Y - \underset{\displaystyle |}{\overset{R_4}{\overset{|}{N}}} - R_1 - O - \left[ R_1 - O \right]_n - R_1 - \overset{R_4}{\overset{|}{N}} - Y - P_1$$

(15) $$C - \left[ CH_2 - O - \left( -R_1 - O - \right)_n - R_1 - O - Y - P_1 \right]_4$$

(16) $$C - \left[ -CH_2 - \underset{R_4}{\underset{|}{N}} - R_1 \left( -O - R_1 \right)_n O - R_1 - \underset{R_4}{\underset{|}{N}} - Y - P_1 \right]_4$$

(17) $$R_5 - N - \left[ R_6 - O - \left( -R_1 - O - \right)_n - R_1 - O - Y - P_1 \right]_x$$

(18) $$P_1 - Y - O - \left[ R_1 - O \right]_m R_1 - O - \left[ R_2 - O \right]_n R_2 - O - Y - P_1$$

(19) $$P_1 - Y - \overset{R_4}{\overset{|}{N}} - R_1 - \left[ O - R_1 \right]_m O - \left[ R_2 - O \right]_n R_2 - \overset{R_4}{\overset{|}{N}} - Y - P_1$$

(20) $$C-\left[-CH_2-O-\left(-R_1-O-\right)_m-R_1-O-\left(-R_2-O-\right)_n-R_2-O-Y-P_1\right]_4$$

(21) $$C-\left[-CH_2\underset{\displaystyle R_4}{\underset{|}{N}}-R_1-\left(-O-R_1-\right)_m-O-\left(-R_2O\right)_n-R_2-\underset{\displaystyle R_4}{\underset{|}{N}}-Y-P_1\right]_4$$

(22) $$R_5-N-\left[-R_6O-\left(-R_1-O-\right)_m-R_1-O-\left(-R_2-O-\right)_n-R_2O-Y-P_1\right]_x$$

In formulas 13-22, each Y is independently selected from the group consisting of amino acids and peptides, and $P_1$, $R_1$, $R_2$, $R_4$, $R_5$, $R_5$ and x are as defined above; and m and n are at least 1; and m and n are sufficiently large that the molecular weight of the polymer is at least 10,000.

Amine terminated versions of Formulas 5, 10 and 22 can also be used, i.e., Formulas 5, 10, and 22 can be modified by replacing the terminal oxygens proximate to $P_1$ with N-$R_4$.

These and other features of the present invention will become better understood from the following description and appended claims.

The present invention is directed to compositions suitable for forming anti-fouling coatings on medical devices, and the method of their use. These compositions can be synthesized from a mixture of:

(a) a poly(alkylene oxide) based polymer, such as polyethylene oxide, terminated with hydroxy or amine groups, and having a molecular weight of at least 10,000;

(b) a precursor of a photochemically reactive or thermochemically reactive moiety such as a halogenated methylbenzophenone or a halogenated acidonitrophenyl;

(c) a strong base such as sodium hydride; and

(d) a non-reactive organic solvent such as tetrahydrofuran or toluene.

The term "non-reactive" indicates that the solvent will not deleteriously react with any of the components of the polymerization reaction.

The poly(alkylene oxide) is modified by alkylation or acylation with a reactive compound to yield a difunctional poly(alkylene oxide). This modified polymer is then applied on a medical device. When the coating is exposed to ultraviolet light or heat, the modified polymer polymerizes and crosslinks to form an anti-fouling coating.

1. Poly(alkylene oxide) Based Polymer

Poly(alkylene oxide) is a generic name given to a class of materials which contain an alkylene oxide repeat unit. The repeating unit can be methylene, ethylene, propylene, butylene, and any other hydrocarbon containing at least one carbon atom, and typically can contain up to about 10 carbon atoms.

For example, the poly(alkylene oxide) based compound can be a polyethylene oxide, having an ethylene oxide repeat unit, with the ends terminated by hydroxy groups. An exemplary compound has the structure:

(23) $$H-O-\left[CH_2-CH_2-O\right]_n-CH_2-CH_2-OH$$

The poly(alkylene oxide) can also be terminated at both ends with amine ($NH_2$) moiety instead of a hydroxy moiety. An exemplary compound has the structure:

(24) 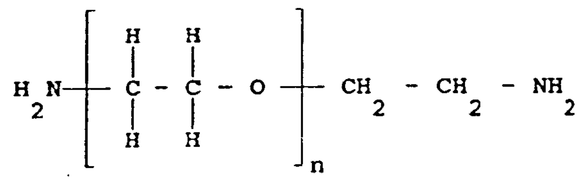

Penta-erythritol or its derivatives can be used as a building block to produce a modified polymer. An exemplary compound based on penta-erythritol has the formula:

$$(25)\qquad C-\left[CH_2O-\left(CH_2-CH_2-O\right)_n-P_1\right]_4$$

Secondary and tertiary amines can be used as a building block to produce poly(alkylene oxide) based polymers. Exemplary of such compound is:

(26) 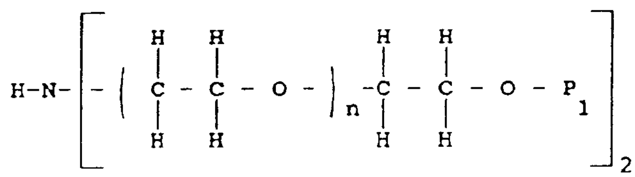

In Formulas 23-26, n is sufficiently large that the average molecular weight of the polymer is at least 10,000.

Preferably the poly(alkylene oxide) based polymer has an average molecular weight of less than 100,000, a preferred molecular weight being approximately 20,000.

The poly(alkylene oxide) based polymer can contain more than one type of alkyl group, such as the class of materials known as POLOXAMERS™, having the formula:

$$(27)\qquad HO-\left[R_1-O\right]_m-R_1-O-\left[R_2-O\right]_n-R_2-O-H$$

These poly(alkylene oxide) based polymers containing two or more blocks of alkylene oxide groups can be used to produce the modified polymers of Formulas 6-10 and 18-22.

Amine terminated poly(alkylene oxides) containing more than one type of alkyl group also can be used, represented by the formula:

(28) 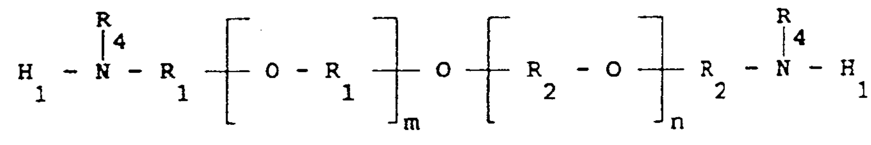

In Formulas 27-28, $R_1$ and $R_2$ are alkyl moieties, containing at least one carbon atom, $R_1$ is different from $R_2$, n is at least 1 and m is at least 1, $R_4$ is as defined above, and n and m are sufficiently large that the average molecular weight of the polymer is at least 10,000. For example, the structure of the polymer depicted in Formula 27 can correspond to a poly(oxyethylene-oxypropylene), where the ethylene to propylene ratio is from about 1:1 to about 3:1.

An example of amine terminated poly(alkylene oxides) suitable for preparing the polymers of Formulas 7, 24 and 28, are marketed under the tradename "JEFFAMINE" by Texaco Chemical Co., Houston, Texas.

2. The Reactive Compound $P_1$

The reactive group is typified by thermochemical groups and photochemical groups, as described and exemplified in U.S. Patent No. 3,959,078 (Guire), the teachings of which are incorporated herein by reference.

The photochemically reactive groups (the covalent bonding of which are activated by actinic radiation) can be aryl, alkyl and acyl azides, oxazidines, isocyanates (nitrene generators), alkyl and ketodiazo derivatives and diazirines (carbene generators), aromatic ketones (triplet oxygen generators), aromatic diazonium derivatives and numerous classes of carbonium ion and radical generators. Reference is made to Frederick J. Darfler and Andrew M. Tometsko, Chapter 2 of Chemistry and Biochemistry of Amino Acids, Peptides and Proteins (Boris Weinstein, ed) Vol. 5, Marcel Dekker, Inc. New York, 1978, for further description of photochemically reactive groups. Azidonitrophenyls, fluoroazido nitrobenzenes, and aromatic ketones form a preferred group due to their stability to chemical reaction conditions in the dark and their susceptibility to activation by light of wave lengths harmless to most biomaterials, to form short-lived reactive intermediates capable of forming covalent bonds in useful yield with most sites on the biomaterial.

Nitrophenylazide derivatives appropriate for use as photochemically reactive groups for the most part can be derived from fluoro-2-nitro-4-azidobenzene, and include 4-azido-2-nitrophenyl(ANP)-4-aminobutyryl, ANP-6-aminocaproyl, ANP-11-aminoundecanoyl, ANP-glycyl, ANP-aminopropyl, ANP-mercaptoethylamino, ANP-diaminohexyl, ANP-diaminopropyl, and ANP-polyethylene glycol. (ANP-6-aminocaproyl, ANP-11-aminoundecanoyl, and ANP-polyethylene glycol are preferred.) Aromatic ketones preferred for use as photochemically reactive groups include benzylbenzoyl and nitrobenzylbenzoyl.

Thermochemical reactive groups (that are activated by heat energy) appropriate for use, are typified by and include diene-dienophile combinations, carbonyl groups, hydroxyl groups, amino groups (1°, 2°), epoxides, thiols, isocyanates, halides, imidates, isothiocyonates, acetylenes, maleimides, diazo compounds, and salts of sulfonyl hydrazones.

The reactive compound can be photochemically reactive, or thermochemically reactive, with photochemically reactive preferred. The photochemically reactive group allows the modified polymer to crosslink upon exposure to ultraviolet radiation.

A preferred precursor for the photochemically reactive compound is halogenated methylbenzophenone having the formula:

(29)

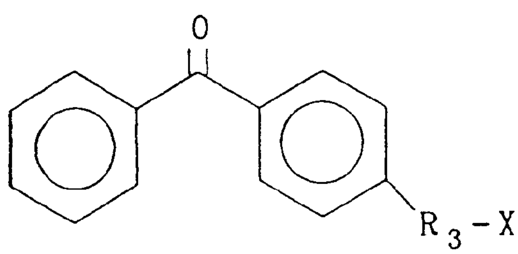

where $R_3$ is an alkyl group or alkyl group having from 1 to about 6 carbon atoms, and X is a halogen moiety.

Halogenated acidonitrophenyl can also be used, having the structure:

(30)

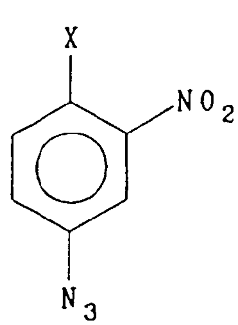

where X is a halogen moiety.

The photochemically reactive groups are incorporated into the polymer by alkylation or acylation to yield a multifunctional poly(alkylene oxide), which upon exposure to ultraviolet light undergoes crosslinking to form a tenacious, lubricous, hydrophilic, anti-fouling material.

During the alkylation process, the halogen moiety is displaced from the halogenated compounds of Formulas 15 and 16, so that the polymer contains the reactive groups, $P_1$, indicated by Formulas 5 and 6.

3. Strong Base

The reactivity of the poly(alkylene oxide) is modified by the addition of a strong base to generate a more nucleophilic species. Examples of these bases include sodium hydride, sodium hydroxide, potassium hydride, and potassium hydroxide.

4. Organic Solvent

A number of organic solvents, inert to reactants, can be used as a medium for the reaction. Solvents such as tetrahydrofuran, toluene, glyme and diglyme can be used.

5. Surfaces for Application

The solid surface that is rendered biocompatible in accordance with the invention desirably is of a synthetic or natural material that is insoluble in physiological fluids. The surface can be one or more surfaces of devices intended to function in contact with tissue and/or fluids of living organisms. The solid surface of the device can be any suitable metal such as polished titanium or stainless steel; a polymer such as polyurethane, silicone elastomers, polyethylene, polytetrafluoroethylene, poly (p-phenyleneterephthalamide), polyvinyl chloride, polypropylene, polyesters, polyacrylates (including polymethacrylates); minerals or ceramics such as hydroxyapatite; human tissue such as bone, skin and teeth; organic materials such as wood, cellulose and compressed carbon; and other natural and synthetic materials such as glass, rubber, wood and the like. Examples of devices which can be provided with biocompatible surfaces in accordance with this invention include vascular graft tubing, dialysis tubing or membrane, blood oxygenator tubing or membrane, ultrafiltration membrane, intra aortic balloons, blood bags, catheters, suture materials, soft or hard tissue prostheses, synthetic prostheses, artificial organs, and lenses for the eye such as contact and intraocular lenses.

The solid surface is desirably thermochemically unreactive. "Thermochemically unreactive" means that the surface is free of any surface treatment designed to increase the ability of the surface to thermochemically react. Examples of thermochemically unreactive surfaces include polytetrafluroethylene, polyethylene, polypropylene, polyvinyl chloride, polyvinylpyrrolidone, silicone elastomers, stainless steel and titanium.

6. Biodegradable Coatings

For the preparation of biodegradable coatings, it is desirable to incorporate in the polymer backbone, a moiety that allows hydrolysis of the coating. Suitable ingredients include amino acids such as alanine, valine, leucine, proline, methionine, aspartic acid, threonine, serine, glutamic acid, glycine, cysteine, phenylalanine, lysine, histidine, argine and aminobutyric acid. Peptide sequences such as arginine-glycine-aspartic acid can also be used.

The biodegradable coating can be used to allow the slow release of a contained drug or other therapeutic agent. Exemplary of such agents are an antimicrobial agent such as penicillin; an anti-thrombogenic agent such as heparin; an anti-inflammatory agent such as dexamethasone sodium phosphate; and a variety of enzymes.

Formulas 7, 8, 9 and 10 show polymers suitable for forming biodegradable coatings.

7. Structures of Modified Polymers

When a hydroxy-terminated poly(alkylene oxide) is substituted with a photochemically or thermochemically reactive moiety, the modified polymer can have the structure shown by Formulas 1 and 6 above. An exemplary modified polymer has the structure:

(31)

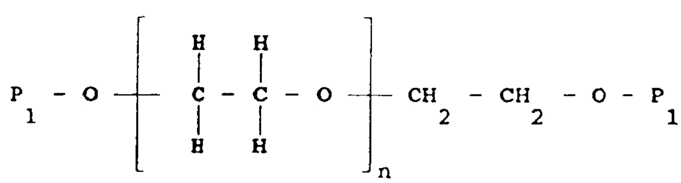

When an amine-terminated poly(alkylene oxide) is reacted with a photochemically or thermochemically reactive compound, the modified polymer has the structure of Formulas 2 and 7 above. An exemplary polymer has the structure:

(32) 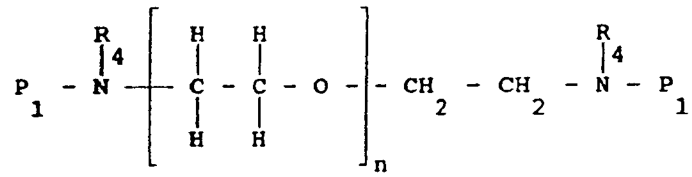

When pentaerythritol or its derivatives are used as building blocks, the modified polymer can be the structure shown by Formula 3 and 4. An exemplary pentaerythritol based polymer has the structure:

$$(33) \qquad C-\left[CH_2O\left(-CH_2CH_2O-\right)_n-CH_2-CH_2-O-P_1\right]_4$$

When dialkyl amino or trialkyl amino compounds, or their derivatives are used as building blocks, poly(alkylene oxides) with multipendant or star configurations can be prepared, as shown by Formula 5.

In these formulas, n is sufficiently high that the average molecular weight of the polymer is at least 10,000, and $P_1$ is a photochemically or thermochemically reactive moiety, and $R_4$ is as defined above.

8. Preparation of Modified Polymers

The following procedure can be used to prepare modified polymers or precursors from a mixture of reactants.

A poly(ethylene oxide) polymer or other poly(alkylene oxide) based polymer is dissolved in toluene or other appropriate solvent and any water present is azeotropically removed. After water removal, dry tetrahydrofuran is added and the mixture is heated to 50 to 60°C in a dry inert atmosphere of nitrogen or argon for a sufficient time, generally about 1 hour, while stirring, to dissolve the polymer.

A strong base, preferably NaH, is then added to the polymer solution in a stoichiometric amount (two moles of base per one mole of polymer). Approximately 30 minutes later, after the dissolution of the base, a reactive group, such as photochemically reactive 4-bromomethylbenzophenone (BBE), in an amount of two moles per one mole of polymer, is added and stirred overnight.

The reaction is sampled at various intervals and the reaction sample is quenched with the acetic acid and the product equivalent weight determined by ultraviolet spectroscopy. The equivalent weight of the modified poly(alkylene oxide) samples can be calculated from corresponding ultraviolet absorbance using the expression:

$$A = cb$$

where A is the molar absorptivity, c is the concentration, and b is the path length.

After quenching the reaction, the solvents are evaporated.

The residue from the reaction is then dissolved in toluene, centrifuged and filtered. The clear solvent solution is then evaporated to remove approximately 70% by volume of the toluene and the solution is then added to cold (0°C) stirred $Et_2O$ to effect precipitation. The product is then collected by filtration.

The reaction that occurs proceeds as follows:

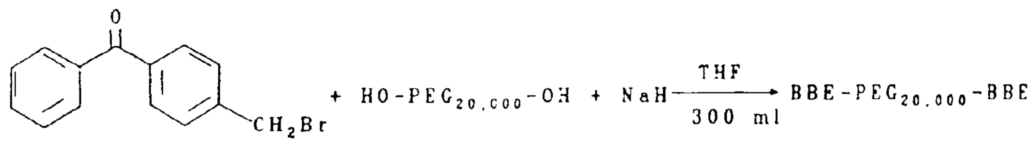


| | | | |
|---|---|---|---|
| 275 g/mole | 20,000 g/mole | 24 g/mole | 20,388 g/mole |
| 5.78 g | 140 g | 0.504 g (.63g of 80%) | 42.7 g (theory) |
| 21.0 mMole (theory) | 7.0 mMole | 21.0 mMole | 7.0 mMole |

where PEG is polyethylene glycol.

9. Applications of the Invention

Because the coating is applied as an aqueous solution followed by a thermal or photo-initiated curing or crosslinking step, the coating can be applied to a wide variety of biomedical devices. Various applications include:

- catheter coatings to increase biocompatibility and lubricity; e.g., urinary tract catheters, coronary catheters, tracheal catheters.
- blood contact device coatings to decrease thrombogenicity; e.g., vascular grafts, shunts, pacemaker leads.
- coatings of subcutaneous or intramuscular implants to minimize fibrous capsule formation; e.g. reconstructive/prosthetic implants, i.e., breast protheses.
- coatings to increase lubricity of the surface; e.g. condoms, pacemaker leads, urinary tract catheters.
- coatings to deliver therapeutics to modify cellular responses and control infection; e.g., pacemaker electrodes and lead bodies for the management of inflammation, defibrillator patch leads and pulse generators for the control of infection.
- lubricous coatings for the delivery of antivirals, spermicides, flavorings or scents; e.g., condoms (male and female).
- a material to be used in the formation of ultraviolet laser induced microcapsules for the controlled release of a variety of therapeutics.

A coating is applied in conventional manners such as by dipping, spraying and brushing. The coating can be effective when applied in thicknesses as thin as 0.01 microns, but generally it is preferably applied in a thickness of at least 1 micron. The thicknesses used herein refer to the thickness before hydration, i.e., before the coating is placed in contact with body fluids.

These and other features of the present invention will become apparent from the following examples.

Example 1

(Preparation of Polymer)

140 g of poly(ethylene oxide) (average molecular weight of 20,000) was dissolved in 1 liter of toluene and the water was azeotropically removed using a Dean Stark trap. The remaining solvent was then removed using a rotary evaporator. 300 ml of dry tetrahydrofuran was added and the mixture was heated to dissolve the PEG. The reaction was kept under a dry inert atmosphere of nitrogen while stirring until the reaction was completed.

0.504 g of sodium hydride (NaH) was added to the stirring solution and after thirty minutes, 5.78 g of 4-bromomethylbenzophenone was added, and the reaction stirred overnight.

The reaction was sampled intermittently to determine the equivalent weight, before quenching the reaction, by the following procedure. A 5 ml sample of the reaction was treated with 50 μl acetic acid and evaporated. Toluene (10 ml) was added to the residue and removed using a rotary evaporator. The residue was dissolved in toluene (10 ml) and filtered with a 0.45μ syringe filter. The clear toluene solution was added to cold $Et_2O$ with stirring. The solid was collected on a Büchner funnel and reprecipitated from toluene (10 ml) and $Et_2O$ (50 ml). The solid was isolated by filtration. Traces of solvents were removed by agitation of the solid at room temperature under a vacuum of less than 1 mm. A solution of the solvent free modified poly(ethylene oxide) in water was made at a concentration of 0.3 to 0.5 mg/ml. The equivalent weight was calculated from the equation:

Eq. Wt. = [Conc(mg/ml)$*$ $\in$ ($1.802*10^4$)]/Absorbance(260 nm)

Once the equivalent weight was below 10,500, the reaction was quenched by addition of 2 moles acetic acid per mole of NaH. The quenched reaction was then rotary evaporated to remove the tetrahydrofuran. The residue was dissolved in toluene (1000 ml), and the warm cloudy toluene solution was centrifuged and filtered to remove insolubles. The clear toluene solution was rotary evaporated to remove 700 ml of the toluene. The warm concentrated toluene solution of the product was then added to 1500 ml of cold (0°C) stirred $Et_2O$. The modified poly(ethylene oxide) was isolated on a Buchner funnel, and reprecipitated from toluene (300 ml) and $Et_2O$ (1500 ml). The final weight of the modified poly(ethylene oxide) obtained was 136g (95% of theory).

Example 2

(Preparation of Anti-fouling Coatings)

The following procedure was be used to coat surfaces with the photochemically reactive polymer of Example 1.

Polyurethene tubing to be coated was cleaned by wiping with a solvent such as isopropyl alcohol or other cleaning agent and then treated in plasma to activate its surface. The plasma treatment involved exposing the surface to argon gas plasma at 250 mTorrs pressure for 2 minutes per side at 250 watts power. The plasma treated tubing was then dip-coated in an aqueous solution of the modified poly(alkylene oxide) of Example 1. The solution which can contain polymer in amounts from 50 to 500 mg/ml, with higher concentrations yielding thicker coatings, was used to coat 3 segments of tubing. The dip consisted of a 30-second soak followed by removal of the device at a rate of approximately 70-80 cm/min. Excess polymer on the surface of the device was drained by allowing the tube to hang for about 15 seconds. The wet samples were then exposed to ultraviolet light for about three minutes in an ELC lamp chamber. The ultraviolet light exposure crosslinked the prepolymer to form a tenacious, lubricous, hydrophilic anti-fouling coating on the surface.

Coating thickness was measured on the three samples prepared from three different concentrations of the BBE-$PEG_{20,000}$-BBE of Example 1, 50 mg/ml, 100 mg/ml, and 200 mg/ml. The coating prepared from 50 mg/ml solution yielded a uniform, dry coating with a thickness the order of 0.5 micron, at 100 mg/ml the dry coating thickness was 1.4 microns, and at 200 mg/ml the dry coating thickness was 3 microns.

Example 3

(Lubricity of the Coating)

The coefficient of friction for the anti-fouling coating on the polyurethane tubing of Example 2 was determined by utilizing the "beef casing" method. This method determines the minimum force required to pull a beef casing across the hydrated polymer surface. For the uncoated polyurethane tubing the average force required was 225.20 grams to yield an average coefficient of friction of 1.11. Hydrated coated tubing yielded an average force of 42.04 (grams) and an average coefficient of friction of 0.21. This represents an increase in lubricity by more than a factor of five over uncoated polyurethane tubing.

Although the present invention has been described in considerable detail with regard to certain preferred versions thereof, other versions are possible. For example, in branch type formulas such as those of Formulas 3, 4, 8, 9, 15, 16, 20, and 21 where there are 4 chains extending from a central carbon atom, or Formulas 5, 10, and 22, where there are 2 or 3 chains extending from a central carbon atom, the chains need not be the same, i.e., the $R_1$'s and $R_2$'s can be different.

Therefore, the spirit and scope of the appended claims should not be limited to the description of the preferred versions contained herein.

## Claims

1. A water-soluble, biocompatible polymer suitable for crosslinking, the polymer comprising at least one poly(alkylene oxide) segment having at least 2 repeating -$R_1$-O- groups, where $R_1$ is an alkyl group containing at least 1 carbon atom, the polymer having at least two terminal photochemically or thermochemically reactive moieties, $P_1$, capable of undergoing crosslinking on photoactivation or heating to form a hydrophilic, lubricous and tenacious coating that resists fouling, the polymer having an average molecular weight of at least 10,000.

2. The polymer of claim 1 having the formula:

$$P_1 - O - \left[ R_1 - O \right]_n - R_1 - O - P_1$$

where n is sufficiently large that the average molecular weight of the polymer is at least 10,000.

3. The polymer of claim 1 having the formula:

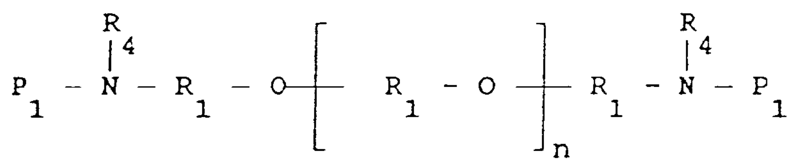

where $R_4$ is selected from the group consisting of hydrogen, hydrocarbon groups containing up to about 10 carbon atoms, and heteroatom-containing hydrocarbon groups containing up to about 10 carbon atoms, and

where n is sufficiently large that the average molecular weight of the polymer is at least 10,000.

4. The polymer of Claim 2, wherein $P_1$ is a photochemically reactive moiety.

5. The polymer of Claim 3, wherein $P_1$ is a photochemically reactive moiety.

6. The polymer of Claim 2 or 3, wherein $R_1$ contains up to 10 carbon atoms.

7. The polymer of Claim 2 or 3, wherein n is sufficiently large that the average molecular weight of the polymer is about 20,000.

8. The polymer of Claim 2 or 3, wherein $R_1$ contains two carbon atoms.

9. The polymer of claim 1 having the formula:

$$P_1 - Y - O - \left[ R_1 - O \right]_n - R_1 - O - Y - P_1$$

where Y is selected from the group consisting of amino acids and peptides, and

where n is sufficiently large that the average molecular weight of the polymer is at least 10,000.

10. The polymer of claim 1 having the formula:

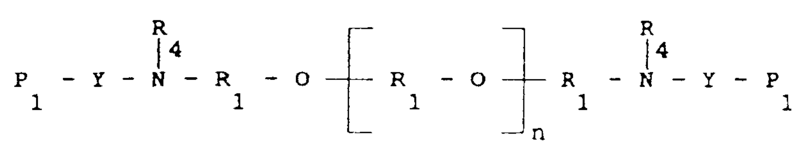

where Y is selected from the group consisting of amino acids and peptides so that the polymer is biodegradable,

where $R_4$ is selected from the group consisting of hydrogen, hydrocarbon groups containing up to about 10 carbon atoms, and heteroatom-containing hydrocarbon groups containing up to about 10 carbon atoms, and

where n is sufficiently large that the average molecular weight of the polymer is at least 10,000.

11. The polymer of claim 1 having the formula:

$$C-\left[-\ CH_2-O-\left(-R_1-O-\right)_n-R_1-O-P_1\right]_4$$

where n is sufficiently large that the average molecular weight of the polymer is at least 10,000.

**12.** The polymer of claim 1 having the formula:

$$C-\left[-\ CH_2-\underset{R_4}{\underset{|}{N}}-R_1-\left(-O-R_1-\right)_n-O-R_1-\underset{R_4}{\underset{|}{N}}-P_1\right]_4$$

where $R_4$ is selected from the group consisting of hydrogen, hydrocarbon groups containing up to about 10 carbon atoms, and heteroatom-containing hydrocarbon groups containing up to about 10 carbon atoms, and

where n is sufficiently large that the average molecular weight of the polymer is at least 10,000.

**13.** The polymer of claim 1 having the formula:

$$R_5-N-\left[-R_6-O-\left(-R_1-O-\right)_n-R_1-O-P_1\right]_x$$

where x is 2 or 3, and for x of 2, $R_5$ is selected from the group consisting of hydrogen, hydrocarbon groups containing up to about 10 carbon atoms, and heteroatom-containing hydrocarbon groups containing up to about 10 carbon atoms, and for x of 3, $R_5$ is excluded,

where n is sufficiently large that the average molecular weight of the polymer is at least 10,000, and

where $R_6$ is an alkyl group containing up to 10 carbon atoms.

**14.** The polymer of claim 1 having the formula:

$$C-\left[CH_2-O-\left(-R_1-O-\right)_n-R_1-O-Y-P_1\right]_4$$

where n is sufficiently large that the average molecular weight of the polymer is at least 10,000, and

where Y is selected from the group consisting of amino acids and peptides so that the polymer is biodegradable.

**15.** The polymer of claim 1 having the formula:

$$C-\left[-CH_2-\underset{\displaystyle R_4}{\underset{|}{N}}-R_1\left(-O-R_1\right)_n O-R_1-\underset{\displaystyle R_4}{\underset{|}{N}}-Y-P_1\right]_4$$

where $R_4$ is selected from the group consisting of hydrogen, hydrocarbon groups containing up to about 10 carbon atoms, and heteroatom-containing hydrocarbon groups containing up to about 10 carbon atoms,

where n is sufficiently large that the average molecular weight of the polymer is at least 10,000, and

where Y is selected from the group consisting of amino acids and peptides so that the polymer is biodegradable.

**16.** The polymer of claim 1 having the formula:

$$R_5-N\left[R_6-O\left(-R_1-O-\right)_n-R_1-O-Y-P_1\right]_x$$

where x is 2 or 3, and for x of 2, $R_5$ is selected from the group consisting of hydrogen, hydrocarbon groups containing up to about 10 carbon atoms, and heteroatom-containing hydrocarbon groups containing up to about 10 carbon atoms, and for x of 3, $R_5$ is excluded,

where n is sufficiently large that the average molecular weight of the polymer is at least 10,000,

where Y is selected from the group consisting of amino acids and peptides so that the polymer is biodegradable, and

$R_6$ is an alkyl group containing up to 10 carbon atoms.

**17.** A water-soluble biocompatible polymer suitable for crosslinking, the polymer comprising at least two polyalkylene oxide segments, a first segment having at least one $-R_1-O-$ unit and a second segment having at least one $-R_2-O-$ unit, where $R_1$ and $R_2$ are alkyl groups having at least 1 carbon atom and $R_1$ and $R_2$ are different from each other, the polymer having at least two terminal photochemically or thermochemically reactive moieties, $P_1$, capable of undergoing crosslinking on photoactivation or heating to form a hydrophilic, lubricous and tenacious coating which resists fouling, the polymer having an average molecular weight of at least 10,000.

**18.** The polymer of claim 17, where $P_1$ is a photochemically reactive moiety.

**19.** The polymer of claim 17, wherein each of $R_1$ and $R_2$ contain up to 10 carbon atoms.

**20.** The polymer of claim 17, having an average molecular weight of about 20,000.

**21.** The polymer of claim 17 having the formula:

$$P_1-O-R_1-\left[-O-R_1-\right]_m-O-\left[-R_2-O-\right]_n-R_2-O-P_1$$

**22.** The polymer of claim 17 having the formula::

$$P_1-\overset{\displaystyle R_4}{\overset{|}{N}}-R_1-\left[O-R_1\right]_m-O-\left[R_2-O\right]_n-R_2-\overset{\displaystyle R_4}{\overset{|}{N}}-P_1$$

where $R_4$ is selected from the group consisting of hydrogen, hydrocarbon groups containing up to about 10 carbon atoms, and heteroatom-containing hydrocarbon groups containing up to about 10 carbon atoms, and

m is at least 1 and n is at least 1, and m and n are sufficiently large that the average molecular weight of the polymer is at least 10,000.

**23.** The polymer of claim 17 having the formula:

$$P_1 - Y - O \left[ R_1 - O \right]_m R_1 - O \left[ R_2 - O \right]_n R_2 - O - Y - P_1$$

where Y is selected from the group consisting of amino acids and peptides so that the polymer is biodegradable, and

where m and n are at least 1 and are sufficiently large that the average molecular weight of the polymer is at least 10,000.

**24.** The polymer of claim 17 having the formula:

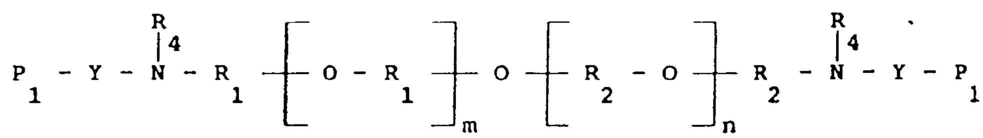

where Y is selected from the group consisting of amino acids and peptides so that the polymer is biodegradable,

where $R_4$ is selected from the group consisting of hydrogen, hydrocarbon groups containing up to about 10 carbon atoms, and heteroatom-containing hydrocarbon groups containing up to about 10 carbon atoms, and

where m and n are at least 1 and are sufficiently large that the average molecular weight of the polymer is at least 10,000.

**25.** The polymer of claim 17 having the formula:

$$C-\left[-CH_2-O\left(-R_1-O-\right)_m-R_1-O\left(-R_2-O-\right)_n-R_2-O-P_1\right]_4$$

where m is at least 1 and n is at least 1, and m and n are sufficiently large that the average molecular weight of the polymer is at least 10,000.

**26.** The polymer of claim 17 having the formula:

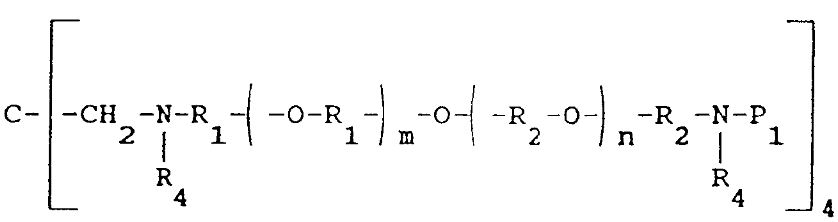

where $R_4$ is selected from the group consisting of hydrogen, hydrocarbon groups containing up to

about 10 carbon atoms, and heteroatom-containing hydrocarbon groups containing up to about 10 carbon atoms, and

m is at least 1 and n is at least 1, and m and n are sufficiently large that the average molecular weight of the polymer is at least 10,000.

**27.** The polymer of claim 17 having the formula:

$$R_5\text{-}N\text{-}\left[-R_6\text{-}O\text{-}\left(\text{-}R_1\text{-}O\text{-}\right)_m\text{-}R_1\text{-}O\text{-}\left(\text{-}R_2\text{-}O\text{-}\right)_n\text{-}R_2\text{-}O\text{-}P_1\right]_x$$

where x is 2 or 3, and for x of 2, $R_5$ is selected from the group consisting of hydrogen, hydrocarbon groups containing up to about 10 carbon atoms, and heteroatom-containing hydrocarbon groups containing up to about 10 carbon atoms, and for x of 3 $R_5$ is excluded, and

where m and n are at least 1, and m and n are sufficiently large that the average molecular weight of the polymer is at least 10,000, and

where $R_6$ is an alkyl group containing up to 10 carbon groups.

**28.** The polymer of claim 17 having the formula:

$$C\text{-}\left[\text{-}CH_2\text{-}O\text{-}\left(\text{-}R_1\text{-}O\text{-}\right)_m\text{-}R_1\text{-}O\text{-}\left(\text{-}R_2\text{-}O\text{-}\right)_n\text{-}R_2\text{-}O\text{-}Y\text{-}P_1\right]_4$$

where m is at least 1 and n is at least 1, and m and n are sufficiently large that the average molecular weight of the polymer is at least 10,000, and

where Y is selected from the group consisting of amino acids and peptides so that the polymer is , biodegradable.

**29.** The polymer of claim 17 having the formula:

$$C\text{-}\left[-CH_2\underset{\substack{|\\R_4}}{N}\text{-}R_1\text{-}\left(\text{-}O\text{-}R_1\text{-}\right)_n\text{-}O\text{-}\left(\text{-}R_2O\right)_m\text{-}R_2\text{-}\underset{\substack{|\\R_4}}{N}\text{-}Y\text{-}P_1\right]_4$$

where $R_4$ is selected from the group consisting of hydrogen, hydrocarbon groups containing up to about 10 carbon atoms, and heteroatom-containing hydrocarbon groups containing up to about 10 carbon atoms,

m is at least 1 and n is at least 1, and m and n are sufficiently large that the average molecular weight of the polymer is at least 10,000, and

where Y is selected from the group consisting of amino acids and peptides so that the polymer is biodegradable.

**30.** The polymer of claim 17 having the formula:

$$R_5\text{-}N\ \text{-}\left[-R_6O\text{-}\left(\text{-}R_1\text{-}O\text{-}\right)_n\text{-}R_1\text{-}O\text{-}\left(\text{-}R_2\text{-}O\text{-}\right)_n\text{-}R_2O\text{-}Y\text{-}P_1\right]_x$$

where x is 2 or 3, and for x of 2, $R_5$ is selected from the group consisting of hydrogen, hydrocarbon groups containing up to about 10 carbon atoms, and heteroatom-containing hydrocarbon groups containing up to about 10 carbon atoms, and for x of 3 $R_5$ is excluded,

where m and n are at least 1, and m and n are sufficiently large that the average molecular weight of the polymer is at least 10,000,

where Y is selected from the group consisting of amino acids and peptides so that the polymer is biodegradable, and

where $R_6$ is an alkyl group containing up to 10 carbon atoms.

**31.** The polymer of any one of Claims 1 or 17, wherein $P_1$ is photochemically reactive and has the formula selected from the group consisting of:

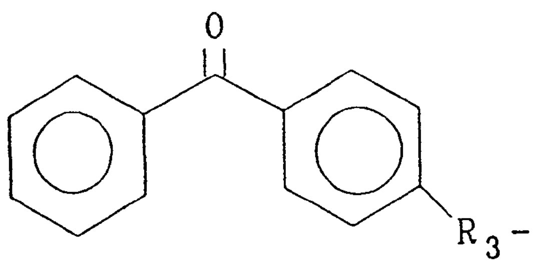

and

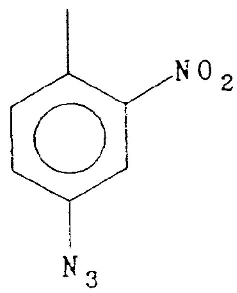

where $R_3$ is selected from the group consisting of alkyl groups, heteroatom-containing alkyl groups containing up to about 6 carbon atoms, acyl groups, alkyl groups with acyl termini, and heteroatom-containing alkyl groups with acyl termini.

**32.** A method for applying a coating that resists fouling to a medical device, the method comprising the steps of:

(a) coating at least a portion of the medical device with an aqueous solution of a polymer, substantially all of the polymer being terminated at both ends with a photochemically reactive group, the molecular weight of the polymer being (i) at least 10,000 and (ii) sufficiently low that the polymer is soluble in water; and

(b) curing the coating by exposing the coating to ultraviolet light.

**33.** A method for applying a coating that resists fouling to a medical device, the method comprising the steps of:

(a) coating at least a portion of the medical device with an aqueous solution of a polymer, substantially all of the polymer being terminated at both ends with a thermochemically reactive group, the molecular weight of the polymer being (i) at least 10,000 and (ii) sufficiently low that the polymer is soluble in water; and

(b) curing the coating by heating the coating.

**34.** The method of claim 32 wherein the polymer comprises at least one poly(alkylene oxide) segment having at least 2 repeating $-R_1-O-$ groups, where $R_1$ is an alkyl group containing at least 1 carbon atom.

**35.** A method for applying a coating that resist fouling to a medical device, the method comprising the steps:
(a) coating at least a portion of the medical device with the polymer of any one of claims 2, 3, 11-13, 17, 21, 22, 25, and 27, where $P_1$ is a photochemically reactive moiety; and
(b) curing the coating by exposing the coating to ultraviolet light.

**36.** The coated medical device prepared by the method of Claim 32 or 34.

**37.** The coated medical device prepared by the method of Claim 35.

**38.** An implantable medical device having a biocompatible coating thereon formed by:
(i) applying to a surface, the polymer of any one of claims 1-5, 11-13, 17, 21, 22, 25, and 27, and
(ii) crosslinking the polymer by exposing the polymer to ultraviolet light, wherein the coating has a thickness of at least 0.01 micron, before hydration.

**39.** The medical device of Claim 36 wherein the medical device is a pacemaker lead or pulse generator.

**40.** The medical device of Claim 37 wherein the medical device is a pacemaker lead or pulse generator.

**41.** The medical device of 36 wherein the medical device is a prophylactic.

**42.** The medical device of Claim 37 wherein the medical device is an ophthalmic lens.

European Patent Office

# EUROPEAN SEARCH REPORT

Application Number
EP 93 30 8174

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | WO-A-86 02087 (YTKEMISKA INSTITUTET)<br>* claims 1,4,17-19 * | 1,2,4, 6-8,31, 32, 34-37, 39-42 | C08G65/32<br>A61L27/00 |
| Y | US-A-4 602 097 (CURTIS J.R.)<br>* column 3, line 7; claim 1 * | 1,2,4, 6-8,31, 32, 34-37, 39-42 | |
| A | EP-A-0 011 237 (BAYER AG)<br>* page 19, line 10; claims 1,5 * | 1-31 | |
| A | EP-A-0 499 836 (BASF AKTIENGESELLSCHAFT)<br>* page 2, line 5 - line 20 *<br>* page 11, line 7 - line 15 * | 1,31 | TECHNICAL FIELDS SEARCHED (Int.Cl.5) |
| A | MAKROMOLEKULARE CHEMIE<br>vol. 187, no. 5 , May 1986<br>pages 1131 - 1144<br>ULBRICH K. ET AL 'Polyethylene glycols containing enzymatically degradable bonds'<br>* page 1132 - page 1132 * | 3,9,10, 14-16 | C08G<br>A61L |

The present search report has been drawn up for all claims

| Place of search | Date of completion of the search | Examiner |
|---|---|---|
| THE HAGUE | 31 January 1994 | O'Sullivan, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)